# EUROPEAN PATENT APPLICATION

(11) **EP 4 811 325 A1**
(43) Date of publication of application: **23.09.2026**
(21) Application number: 26161555.3
(22) Date of filing: 02.03.2026
(51) Int. Cl.: G08B 17/103, G08B 17/113

(54) **SMOKE DETECTION SYSTEM USING A LASER BEAM SPECTRAL PATTERN**

(30) Priority: 19.03.2025 US 202519083702
(71) Applicant: Honeywell International Inc., Charlotte, NC 28202 (US)
(72) Inventor: KOTHARI, Sanjay, Charlotte, 28202 (US)
(74) Representative: Haseltine Lake Kempner LLP

(57) **Abstract**

A smoke detection system using a laser beam spectral pattern is described herein. A smoke detection system includes a laser beam emitter to emit a laser beam, a plurality of reflectors to reflect the laser beam through a monitored area, and a detector configured to receive the reflected laser beam and detect a spectral pattern of the reflected laser beam. The smoke detection system further includes a controller coupled to the detector, wherein the controller is configured to determine whether the spectral pattern of the reflected laser beam indicates that smoke is present in the monitored area and generate an alarm responsive to determining the spectral pattern of the reflected laser beam indicates smoke is present in the monitored area.

## Description

### Technical Field

The present disclosure relates generally to devices, methods, and systems for smoke detection system using a laser beam spectral pattern.

### Background

Large facilities (e.g., buildings), such as commercial facilities, office buildings, hospitals, and the like, may have a fire alarm system that can be triggered during an emergency situation (e.g., a fire) to warn occupants to evacuate. For example, a fire alarm system may include a fire control panel and a plurality of fire sensing devices (e.g., smoke detectors), located throughout the facility (e.g., on different floors and/or in different rooms of the facility) that can sense a fire occurring in the facility and provide a notification of the fire to the occupants of the facility via alarms.

The fire sensing devices of a fire alarm system can include one or more sensors. The one or more sensors can include an optical smoke sensor, a heat sensor, a gas sensor, and/or a flame sensor, for example, that can be used to detect a fire by, for example, detecting the smoke emitted from the fire.

### Brief Description of the Drawings

Figure 1 illustrates a block diagram of a smoke detection system using a laser beam spectral pattern in accordance with an embodiment of the present disclosure.
Figure 2 illustrates an example of a monitored area of a smoke detection system using a laser beam spectral pattern in accordance with an embodiment of the present disclosure.
Figure 3 illustrates spectral patterns of a laser beam in accordance with an embodiment of the present disclosure.
Figure 4 illustrates an example method of operating a smoke detection system using a laser beam spectral pattern in accordance with an embodiment of the present disclosure.

### Detailed Description

A smoke detection system using a laser beam spectral pattern is described herein. One system includes a laser beam emitter to emit a laser beam, a plurality of reflectors to reflect the laser beam through a monitored area, a detector configured to receive the reflected laser beam and detect a spectral pattern of the reflected laser beam, and a controller coupled to the detector, wherein the controller is configured to determine whether the spectral pattern of the reflected laser beam indicates that smoke is present in the monitored area and generate an alarm responsive to determining the spectral pattern of the reflected laser beam indicates smoke is present in the monitored area.

One way in which a fire sensing device (e.g., of a smoke detection system) can detect smoke is by emitting a laser beam which passes through the smoke and detecting a change in a spectral pattern of the laser beam after the laser beam passes through the smoke. However, limitations in the layout of previous smoke detectors have resulted in previous smoke detectors only being able to detect a limited number of changes in the spectral pattern of the laser beam. For instance, previous smoke detectors may only be able to use a reflection of infrared energy to detect smoke, and/or may only be able to detect a certain type of molecule, which can result in false alarms.

In contrast to previous smoke detectors which are configured to only detect a certain type of molecule, smoke detection systems in accordance with the present disclosure include an emitter that can be programmed to emit laser beams with different spectral wavelengths based on the molecules a user wants to detect. Further, in contrast to previous smoke detection systems in which multiple detectors are placed in different locations, smoke detection systems in accordance with the present disclosure can include reflectors to reflect the laser beam through different portions of the monitored area before reflecting the laser beam to a single detector. Smoke detection systems in accordance with the present disclosure can also reduce the occurrence of false alarms caused by other substances, such as dust, in the smoke chamber of conventional smoke detectors since molecules in the smoke are determined based on wavelengths being absorbed by molecules in the smoke.

Accordingly, such smoke detection systems in accordance with the present disclosure allow a user to choose which molecules to measure by programming a laser beam emitter to emit a laser beam that includes a wavelength that can be absorbed by the molecule the user wants to measure. Further, such smoke detection systems in accordance with the present disclosure can cover a wider area of a monitored area of the smoke detection system while using a single detector to receive the laser beam, thereby reducing the cost of the smoke detection systems. Smoke detection systems in accordance with the present embodiment also reduce the occurrence of false alarms caused by other substances, such as dust, in the monitored area since molecules in the smoke are determined based on wavelengths being absorbed by molecules in the smoke.

In the following detailed description, reference is made to the accompanying drawings that form a part hereof. The drawings show by way of illustration how one or more embodiments of the disclosure may be practiced.

These embodiments are described in sufficient detail to enable those of ordinary skill in the art to practice one or more embodiments of this disclosure. It is to be understood that other embodiments may be utilized and that mechanical, electrical, and/or process changes may be made without departing from the scope of the present disclosure.

As will be appreciated, elements shown in the various embodiments herein can be added, exchanged, combined, and/or eliminated so as to provide a number of additional embodiments of the present disclosure. The proportion and the relative scale of the elements provided in the figures are intended to illustrate the embodiments of the present disclosure and should not be taken in a limiting sense.

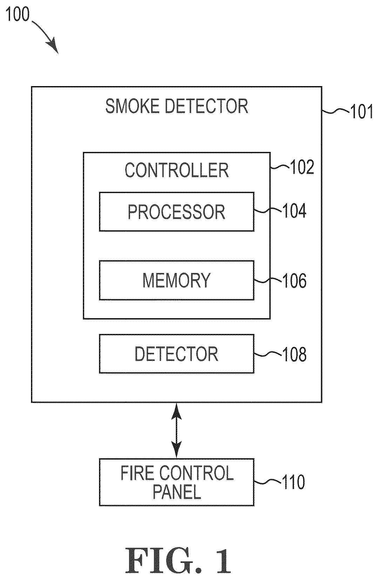

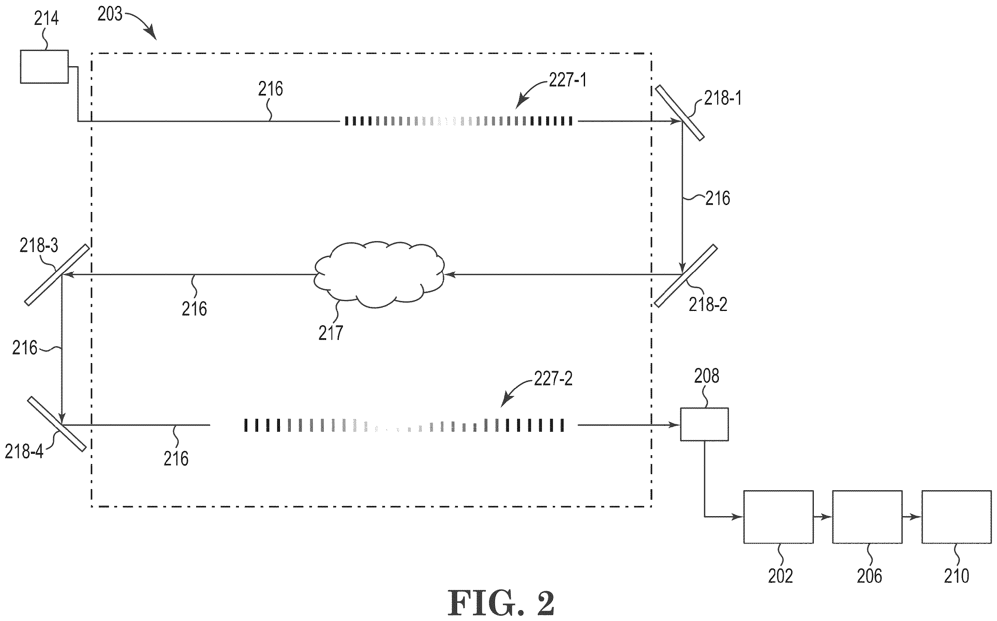

The figures herein follow a numbering convention in which the first digit or digits correspond to the drawing figure number and the remaining digits identify an element or component in the drawing. Similar elements or components between different figures may be identified by the use of similar digits. For example, 103 may reference element "03" in Figure 1, and a similar element may be referenced as 203 in Figure 2.

As used herein, "a," "an," or "a number of" something can refer to one or more such things, while "a plurality of" something can refer to more than one such things. For example, "a number of components" can refer to one or more components, while "a plurality of components" can refer to more than one component.

Figure 1 illustrates a block diagram of a smoke detection system 100 using a laser beam spectral pattern in accordance with an embodiment of the present disclosure. The smoke detection system 100 includes a smoke detector 101 that includes a controller 102, and a detector 108. Detector 108 can be, for example, a photodetector. Controller 102 can be, for instance, a microcontroller. The smoke detection system 100 can also include a fire control panel 110.

Smoke detection system 100 can be part of a fire alarm system for a large facility (e.g., building), such as, for instance, a commercial facility, office building, hospital, or school, and smoke detector 101 and fire control panel can be located at the facility. For instance, fire control panel 110 can be at a fixed location in the facility, and smoke detector 101 can be located in a different location of the facility, such as, for instance, in a particular room, area, or zone of the facility. Further, smoke detection system can include a number of additional smoke detectors analogous to smoke detector 101 each located at different locations (e.g. different rooms, areas, zones, floors, etc.) throughout the facility.

In some embodiments, the smoke detector 101 can be in communication with the fire control panel 110. Smoke detector 101 and fire control panel 110 can communicate via a wired or wireless network, for example. Fire control panel 110 can be used to monitor and/or control components of the fire alarm system of the facility. For instance, a user can use fire control panel 110 to directly control the operation of (e.g., actions performed by) the components of the fire alarm system of the facility. Further, fire control panel 110 can receive (e.g., collect) data, such as, for instance, real-time operational data, from the components of the fire alarm system of the facility. As an additional example, fire control panel 110 can receive signals (e.g., alarm signals) from the components of the fire alarm system of the facility indicating that an emergency event (e.g., a fire) is occurring in the facility. As an additional example, fire control panel 110 can send signals (e.g., commands) to the components of the fire alarm system of the facility to activate during an emergency event (e.g., a fire) occurring in the facility.

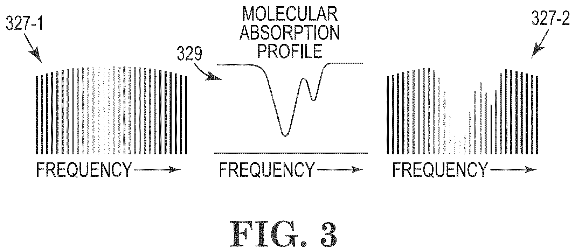

The controller 102 can include a processor 104 and a memory 106. Memory 106 can be any type of storage medium that can be accessed by processor 104 to perform various examples of the present disclosure. For example, memory 106 can be a non-transitory computer readable medium having computer readable instructions (e.g., computer program instructions) stored thereon that are executable by processor 104 to detect a change in the spectral pattern (e.g., spectral patterns 327-1, 327-2 in Figure 3) of a laser beam received by a smoke detector 101 in accordance with the present disclosure. For instance, processor 104 can execute the executable instructions stored in memory 106 to detect a change in the spectral pattern of a laser beam after the laser beam passes through smoke in an area of the facility (e.g., a zone, room, etc.,) being monitored by smoke detection system 100 (e.g., by smoke detector 101). The processor 104 can further execute instructions to determine which molecules are in the smoke and the concentration of the molecules in the smoke based on the change in the spectral pattern.

Memory 106 can be volatile or nonvolatile memory. Memory 106 can also be removable (e.g., portable) memory, or non-removable (e.g., internal) memory. For example, memory 106 can be random access memory (RAM) (e.g., dynamic random access memory (DRAM) and/or phase change random access memory (PCRAM)), read-only memory (ROM) (e.g., electrically erasable programmable read-only memory (EEPROM) and/or compact-disc read-only memory (CD-ROM)), flash memory, a laser disc, a digital versatile disc (DVD) or other optical storage, and/or a magnetic medium such as magnetic cassettes, tapes, or disks, among other types of memory.

Further, although memory 106 is illustrated as being located within smoke detector 101, embodiments of the present disclosure are not so limited. For example, memory 106 can also be located internal to another computing resource (e.g., enabling computer readable instructions to be downloaded over the Internet or another wired or wireless connection).

As an example, detector 108 can receive a laser beam that has passed through the monitored area. For instance, the laser beam can be reflected through the monitored area, as will be further described herein (e.g., in connection with Figure 2). The processor 104, which can receive a signal from the detector 108, can determine whether a spectral pattern of the received laser beam indicates that smoke is present in the monitored area based on whether the spectral pattern has changed while passing through the monitored area. For instance, a change in the spectral pattern of the laser beam can indicate that the laser beam passed through smoke within the monitored area. In some embodiments, the molecules within the smoke can absorb a portion of the spectral pattern of the laser beam, which can cause the change in the spectral pattern. After the smoke molecules have absorbed a portion of the spectral pattern, the remaining portion of the spectral pattern can be analyzed by the controller 102 after the detector 108 receives the laser beam. By analyzing characteristics of the spectral pattern of the laser beam after the laser beam has passed through the smoke, the controller 102 can determine which type of molecules are present in the smoke and/or the concentration of those molecules.

In some embodiments, the laser beam can be a dual comb laser beam. As used herein, the term "dual comb laser beam" refers to a laser beam that uses two distinct spectral patterns with slightly different repetition rates. As used herein, the term "repetition rates" refers to the number of pulses a laser beam emits per second. The laser beam can be a dual comb laser beam because dual comb laser beams include the 100 line spectral patterns needed to accurately measure the concentration of molecules in the smoke. Due to the laser beam being a dual-comb laser beam, both combs can include different wavelengths and therefore experience different changes due to the wavelengths being absorbed by the molecules in the smoke. This can affect the shape of the spectral pattern of the laser beam. The laser beam can be emitted as either a pulse (e.g., a series of pulses) or continuously.

After analyzing the laser beam, the controller 102 can determine which type of molecules are present in the smoke based on the change in the spectral pattern of the laser beam. The controller 102 can further determine whether a concentration of molecules in the smoke is above a threshold concentration of molecules. In some embodiments, responsive to determining the concentration of molecules is above a threshold concentration of molecules, the controller 102 can send an alarm signal to an alarm within the smoke detector 101 (e.g., to activate the alarm). The alarm can be, for example, an audio alarm and/or a visual alarm. In some embodiments, responsive to determining the concentration of molecules in the smoke is above a threshold concentration of molecules, the controller can send an alarm signal to a fire control panel 110 coupled to the smoke detector 101.

Figure 2 illustrates an example of a monitored area 203 of a smoke detection system (e.g., smoke detection system 100 previously described in connection with Figure 1) using a laser beam spectral pattern in accordance with an embodiment of the present disclosure. As used herein, the term "monitored area" refers to an area through which the laser beam 216 travels before the detector 208 receives the laser beam 216. In some embodiments, the monitored area 203 can be a portion of a building such as a zone, room, or floor of the building, including, for instance, the ceiling of a zone, room, or floor in the building.

The smoke detection system can include a laser beam emitter 214, which can emit a laser beam 216 through the monitored area 203. In some embodiments, as stated previously, the laser beam 216 emitted from the laser beam emitter 214 can be a dual comb laser beam.

The smoke detection system can include a plurality of reflectors 218-1, 218-2, 218-3, 218-4 (individually or collectively referred to as reflectors 218) positioned and/or arranged to reflect the laser beam 216 through the monitored area 203. For instance, as shown in Figure 2, the laser beam emitter 214 can emit the laser beam 216 through the monitored area 203 to a first reflector 218-1, the first reflector 218-1 can reflect the laser beam 216 to a second reflector 218-2, the second reflector 218-2 can reflect the laser beam 216 through the monitored area 203 to a third reflector 218-3, the third reflector 218-3 can reflect the laser beam 216 to the fourth reflector 218-4, and the fourth reflector 218-4 can reflect the laser beam 216 through the monitored area 203 to a detector 208 (which can be, for example, detector 108 previously described in connection with Figure 1).

In some embodiments, the reflectors 218 can be located within the monitored area on the sidewalls of the monitored area. For instance, as shown in Figure 2, the first reflector 218-1 and the second reflector 218-2 can be on a first sidewall of the monitored area 203 and the third reflector 218-3 and the fourth reflector 218-4 can be on a second sidewall of the monitored area 203 that is different than (e.g., opposite from) the first sidewall. Further, as illustrated in Figure 2, smoke 217 may not fill the entire monitored area 203. Therefore, the laser beam 216 can pass through a portion of the monitored area 203 without passing through smoke 217 but pass through smoke 217 while passing through a different portion of the monitored area 203. For instance, first reflector 218-1 may reflect laser beam 216 to second reflector 218-2 before the laser beam passes through smoke 217, and third reflector 218-3 may reflect laser beam 216 to fourth reflector 218-4 after the laser beam passes through smoke 217, as illustrated in Figure 2.

The detector 208 can comprise a photodiode and can receive the laser beam 216 after it has been reflected and detect a spectral pattern of the laser beam 216. A photodiode is a diode that consumes light energy to produce an electrical current. The detector 208 can send an electrical current indicating the spectral pattern of the laser beam 216 to the controller 202 (e.g., controller 102 previously described in connection with Figure 1) that is coupled to the detector 208. The controller 202 can be configured to determine whether the spectral pattern of the reflected laser beam 216 indicates that smoke 217 is present in the monitored area 203 based on a change in the spectral pattern of the reflected laser beam 216. For instance, in the example illustrated in Figure 2, the spectral pattern 227-2 of the laser beam 216 after it has been reflected by reflector 218-4 has changed from the spectral pattern 227-1 of the laser beam 216 before it has been reflected by reflector 218-1 as a result of the laser beam 216 passing through smoke 217, and the changed spectral pattern 227-2 indicates the presence of smoke 217. The controller 202 can analyze the electrical current received from the detector 208 to determine the shape of the spectral pattern of the laser beam received by the detector 208.

The change in the spectral pattern of the reflected laser beam 216 can be caused by the molecules of the smoke 217 absorbing portions of the spectral pattern of the reflected laser beam 216. In some embodiments, the change in the spectral pattern of the laser beam 216 can indicate which type of molecules are present in the smoke 217 because different types of molecules can absorb different wavelengths within the spectral pattern of the laser beam 216.

The controller 202 can be further configured to determine whether a concentration of molecules in the smoke 217 is above a threshold concentration of molecules. The concentration of a certain type of molecule in the smoke can correspond to the amount of a certain wavelength of the spectral pattern of the laser beam 216 that is absorbed. For example, if a higher concentration of a certain type of molecule is present in the smoke 217, more of the wavelength that is absorbed by that molecule is absorbed compared to if there was a lower concentration of that type of molecule in the smoke 217. Accordingly, the concentration of molecules being above the threshold concentration can indicate the presence of smoke 217.

Further, the controller 202 can be coupled to the memory 206 and can be configured to send an alarm signal to a fire control panel 210 (e.g., fire control panel 110 previously described in connection with Figure 1) responsive to determining the presence of smoke 217 (e.g., that the concentration of certain molecules in the smoke 217 is above the threshold concentration of molecules). The fire control panel 210 can be configured to activate an alarm (e.g., by sending a command to the alarm) of the smoke detection system responsive to receiving the alarm signal. Additionally or alternatively, the controller 202 can be configured to send an alarm signal to an alarm in the smoke detection system responsive to determining the presence of smoke (e.g., that the concentration of certain molecules in the smoke is above the threshold concentration of molecules). The alarm in the smoke detection system can be configured to activate in response to receiving the alarm signal.

Figure 3 illustrates spectral patterns of a laser beam (e.g., laser beam 216 previously described in connection with Figure 2) in accordance with an embodiment of the present disclosure. Figure 3 includes spectral patterns 327-1, 327-2 (individually or collectively referred to as spectral patterns 327). In some embodiments, spectral pattern 327-1 can be a spectral pattern of the laser beam before the laser beam passes through smoke (e.g., smoke 217 illustrated in Figure 2) and spectral pattern 327-2 can be a spectral pattern of the laser beam after the laser beam passes through smoke.

As stated previously, the spectral pattern 327 can be the spectral pattern of a dual comb laser beam. A laser beam emitter can be configured to emit each comb of the dual comb laser beam simultaneously. In some embodiments, a first comb of the dual comb laser beam can be spaced 100 nanometers (nm) apart from a second comb of the dual comb laser beam.

Figure 3 further illustrates a molecular absorption profile 329. The molecular absorption profile 329 can illustrate which portions of the spectral pattern 327 have been absorbed by molecules in the smoke. The molecular absorption profile 329 can also illustrate a concentration of certain molecules in the smoke based on the amount of certain wavelengths within the spectral pattern 327 that have been absorbed. In the example illustrated in Figure 3, the shape of the molecular absorption profile 329 corresponds to the change in shape of the spectral pattern 327-2 in comparison to the shape of spectral pattern 327-1.

In some embodiments, a laser beam emitter (e.g., laser beam emitter 214 in Figure 2) can emit a laser beam comprising a spectral pattern 327 composed to detect a specific molecule. For example, to detect carbon dioxide (CO₂), the laser beam emitter can emit a laser beam comprising a spectral pattern that includes wavelengths that would be absorbed by CO₂) molecules. Since the laser beam is a dual comb laser beam, a first comb of a laser beam can be configured to detect a first molecule and a second comb can be configured to detect a second molecule. For example, a first comb of the dual comb laser beam can include wavelengths that would be absorbed by a first molecule and the second comb of the dual comb laser beam can include wavelengths that would be absorbed by a second molecule that is different than the first molecule. This may allow a controller to detect multiple types of molecules within smoke through which the laser beam has passed.

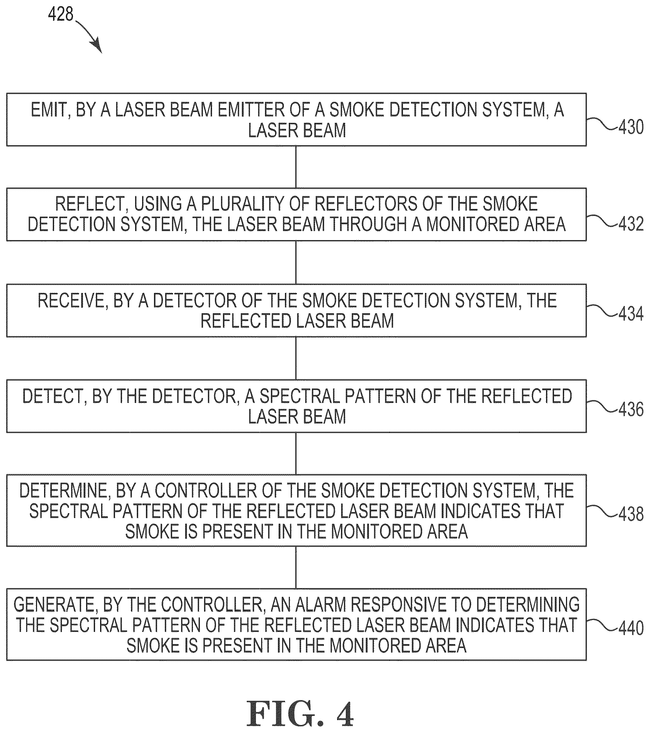

Figure 4 illustrates an example method 428 of operating a smoke detection system using a laser beam spectral pattern in accordance with an embodiment of the present disclosure. In some embodiments, the method illustrated in Figure 4 can be performed by the smoke detection system 100 previously described in connection with Figure 1 (e.g., the controller referred to can be the controller 102 previously described in connection with Figure 1).

At block 430, the method 428 can include emitting, by a laser beam emitter of the smoke detection system, a laser beam. The laser beam emitter and smoke detector can be, for example, laser beam emitter 214 previously described herein. As stated previously, the laser beam can be a dual comb laser beam. Further, the laser beam emitter can be programmed to emit a laser beam that can detect the presence of certain molecules in smoke by including wavelengths in the laser beam that can be absorbed by those types of molecules.

At block 432, the method 428 can include reflecting, using a plurality of reflectors of the smoke detection system, the laser beam through a monitored area. The reflectors can be, for example, reflectors 218 previously described in connection with Figure 2. As stated previously, the reflectors can be placed on the sidewalls of the area monitored by the smoke detection system. In some embodiments, if the smoke does not fill the entire monitored area, the laser beam can pass through certain portions of the monitored area without passing through the smoke but pass through the smoke while traveling through a different portion of the monitored area. For instance, as previously described in the embodiment shown in Figure 2, the laser beam emitter can emit a laser beam that is reflected by a first reflector and then a second reflector without the laser beam passing through the smoke. The laser beam can then pass through the smoke after being reflected from the second reflector to the third reflector. The laser beam can then be reflected from the third reflector to a fourth reflector and then to a detector without passing through the smoke. Hence, the laser beam can be reflected before the laser beam passes through the smoke and after the laser beam passes through the smoke. Embodiments are not limited to the example shown in Figure 2 and whether the laser beam passes through the smoke is dependent on whether smoke is present in the path through which the laser beam is being emitted and reflected.

At block 434, the method 428 includes receiving, by a detector (e.g., detector 108 previously described in connection with Figure 1) of a smoke detector (e.g., smoke detector 101 previously described in connection with Figure 1), the reflected laser beam. In some embodiments, the detector can be a photodiode that converts light into an electrical current.

At block 436, the method 429 can include detecting, by the detector, a spectral pattern of the reflected laser beam. After the detector receives the spectral pattern, the photodiode of the detector can absorb the photons in the spectral pattern and produce an electrical current that is sent to the controller.

At block 438, the method 429 can include determining, by the controller of the smoke detector, that the spectral pattern of the reflected laser beam indicates that smoke is present in the monitored area. For example, the controller can receive the electrical current produced by the detector, and use data stored in a memory of the smoke detector to analyze the electrical current received from the detector and determine a concentration of certain molecules in the smoke through which the laser beam has passed.

At block 440, the method 429 can include generating, by the controller, an alarm responsive to determining the spectral pattern of the reflected laser beam indicates that smoke is present in the monitored area. In some embodiments, the alarm can be generated by sending an alarm signal to either an alarm in the smoke detector or an alarm in the fire alarm panel. The alarm can be an audio alarm, a visual alarm, both, or some other type of alarm.

Although specific embodiments have been illustrated and described herein, those of ordinary skill in the art will appreciate that any arrangement calculated to achieve the same techniques can be substituted for the specific embodiments shown. This disclosure is intended to cover any and all adaptations or variations of various embodiments of the disclosure.

It is to be understood that the above description has been made in an illustrative fashion, and not a restrictive one. Combination of the above embodiments, and other embodiments not specifically described herein will be apparent to those of skill in the art upon reviewing the above description.

The scope of the various embodiments of the disclosure includes any other applications in which the above structures and methods are used. Therefore, the scope of various embodiments of the disclosure should be determined with reference to the appended claims, along with the full range of equivalents to which such claims are entitled.

In the foregoing Detailed Description, various features are grouped together in example embodiments illustrated in the figures for the purpose of streamlining the disclosure. This method of disclosure is not to be interpreted as reflecting an intention that the embodiments of the disclosure require more features than are expressly recited in each claim.

Rather, as the following claims reflect, inventive subject matter lies in less than all features of a single disclosed embodiment. Thus, the following claims are hereby incorporated into the Detailed Description, with each claim standing on its own as a separate embodiment.

## Claims

1. A smoke detection system (100), comprising:
a laser beam emitter (214) to emit a laser beam (216);
a plurality of reflectors (218) to reflect the laser beam (216) through a monitored area (203);
a detector (108, 208) configured to:
receive the reflected laser beam (216); and
detect a spectral pattern of the reflected laser beam (216); and
a controller (102, 202) coupled to the detector (108, 208), wherein the controller (102, 202) is configured to:
determine whether the spectral pattern of the reflected laser beam (216) indicates that smoke (217) is present in the monitored area (203); and
generate an alarm responsive to determining the spectral pattern of the reflected laser beam (216) indicates smoke (217) is present in the monitored area (203).

2. The smoke detection system of claim 1, wherein the controller is configured to determine the spectral pattern of the reflected laser beam indicates that smoke is present in the monitored area based on a change in the spectral pattern of the reflected laser beam.

3. The smoke detection system of claim 2, wherein the change in the spectral pattern of the reflected laser beam is caused by molecules of the smoke absorbing portions of the spectral pattern of the reflected laser beam.

4. The smoke detection system of claim 2, wherein the change in the spectral pattern indicates which type of molecules are present in the smoke.

5. The smoke detection system of any one of claims 1-4, wherein the controller is further configured to determine whether a concentration of molecules in the smoke is above a threshold concentration of molecules.

6. The smoke detection system of claim 5, wherein the controller is configured to send an alarm signal to a fire alarm control panel (110, 210) responsive to determining the concentration of molecules in the smoke is above the threshold concentration of molecules.

7. The smoke detection system of claim 5, wherein the controller is configured to send an alarm signal to an alarm responsive to determining the concentration of molecules in the smoke is above the threshold concentration of molecules.

8. The smoke detection system of claim 7, wherein the alarm is configured to activate in response to receiving the alarm signal.

9. The smoke detection system of any one of claims 1-4, wherein the plurality of reflectors are located on sidewalls of the monitored area.

10. The smoke detection system of any one of claims 1-4, wherein a first reflector 218-1) of the plurality of reflectors reflects the laser beam to a second reflector (218-2) of the plurality of reflectors before the laser beam passes through the smoke.

11. The smoke detection system of claim 10, wherein the second reflector of the plurality of reflectors reflects the laser beam through the smoke to a third reflector (218-3) of the plurality of reflectors.

12. A method, comprising:
emitting, by a laser beam emitter (214) of a smoke detection system (100), a laser beam (216);
reflecting, using a plurality of reflectors (218) of the smoke detection system (100), the laser beam (216) through a monitored area (203);
receiving, by a detector (108, 208) of the smoke detection system (100), the reflected laser beam (216);
detecting, by the detector (108, 208), a spectral pattern of the reflected laser beam (216);
determining, by a controller (102, 202) of the smoke detection system (100), the spectral pattern of the reflected laser beam (216) indicates that smoke (217) is present in the monitored area (203); and
generating, by the controller (102, 202), an alarm responsive to determining the spectral pattern of the reflected laser beam (216) indicates that smoke (217) is present in the monitored area (203).

13. The method of claim 12, wherein the laser beam is a dual comb laser beam.

14. The method of claim 12, wherein the method includes reflecting the laser beam before the laser beam passes through the smoke.

15. The method of claim 12, wherein the method includes reflecting the laser beam after the laser beam passes through the smoke.
